# EUROPEAN PATENT APPLICATION

(11) **EP 4 643 875 A1**
(43) Date of publication of application: **05.11.2025**
(21) Application number: 23912868.9
(22) Date of filing: 26.12.2023
(51) Int. Cl.: A61K 39/385, A61K 39/12, A61K 39/215, A61K 47/69, A61K 48/00, A61K 39/00

(54) **VACCINE COMPOSITION COMPRISING GOLD-NANOPARTICLE-CARRIER HAVING DOUBLE-STRANDED DNA BOUND THERETO**

(30) Priority: 26.12.2022 KR 20220184295
(71) Applicant: NES BIOTECHNOLOGY CO., LTD., Seoul 06974 (KR)
(72) Inventor: LEE, Kangseok, Gwangju-si, Gyeonggi-do 12765 (KR); BAE, Jeehyeon, Gwangju-si, Gyeonggi-do 12765 (KR); YEOM, Jihyun, Yangju-si, Gyeonggi-do 11426 (KR); SIM, Sehoon, Suwon-si, Gyeonggi-do 16493 (KR); KIM, Hongman, Suwon-si, Gyeonggi-do 16438 (KR); RYU, Minkyung, Seoul 07364 (KR); SONG, Wooseok, Seoul 05698 (KR)
(74) Representative: J A Kemp LLP
(86) International application number: PCT/KR2023/021565
(87) International publication number: WO 2024/144193

(57) **Abstract**

The present invention relates to a vaccine composition including double-stranded DNA delivered into cells via gold nanoparticles, and more particularly, to a vaccine composition characterized in that the double-stranded DNA is derived from a viral, bacterial or cancer gene and expresses an antigen to induce an immune response.

## Description

### [Technical Field]

The present invention relates to the use of a gene carrier, which includes gold nanoparticles and a double-stranded DNA bound to the surfaces of the gold nanoparticles, as a vaccine.

### [Background Art]

The technology for delivering a genetic material into cells to produce antigens or proteins for treatment or prevention has advanced significantly in recent years, especially due to the recent pandemic. Accordingly, in addition to antigen vaccines, various gene vaccines such as DNA vaccines, mRNA (messenger RNA) vaccines, and viral vector vaccines are being developed.

DNA is the simplest genetic material that is easily genetically modified, which can shorten the development period for use as a therapeutic agent such as a vaccine. DNA is also very economical in terms of production facility construction and production costs compared to other vaccine candidates such as viruses, proteins, and RNA, and has the advantage of easy storage and distribution because it has excellent stability. However, DNA vaccines require a process in which they are delivered to the nuclei of cells and produce mRNA directly in the nuclei, and vectors such as plasmids are mainly used for delivery. DNA injected into the nuclei may continuously produce mRNA and steadily produce antigen proteins, but may cause side effects such as innate immune responses and the like because other genetic traits are injected into the cell nuclei in our body and transported. In other words, plasmids, which are mainly used to transport DNA, have the risk of side effects because they can transfer bacterial genes. Accordingly, various studies are being conducted on carriers that safely transport genes into cells for the delivery and expression of therapeutic substances. In particular, it is necessary to develop delivery substances that increase transport efficiency and ensure stable expression of therapeutic substances.

In particular, due to the recent outbreak of large-scale infectious diseases such as COVID-19 and influenza, the rapid development of vaccines to respond to infectious diseases has become very important. In particular, in response to the recent COVID-19 pandemic, various types of vaccines such as DNA vaccines and mRNA vaccines as well as existing antigen vaccines have been developed, and various types of delivery vehicles have also been developed to effectively deliver these vaccines. In particular, DNA vaccines have the advantage of ensuring safety compared to mRNA vaccines, which are temperature-sensitive and structurally unstable, but since they must be delivered into the cell nuclei, the development of effective delivery technology is required.

### [Disclosure]

### [Technical Problem]

Accordingly, the present inventors have made extensive research efforts to develop a double-stranded DNA delivery technique to efficiently deliver double-stranded DNA (dsDNA), which has no cytotoxicity and may be expressed within cells, to the nuclei within cells, and have invented a delivery system capable of delivering genes to the nuclei within cells by covalently attaching thiolated double-stranded DNA to the surfaces of gold nanoparticles (AuNPs), and a gene carrier that may be used as a vaccine when the double-stranded DNA is introduced into cells to autonomously express an antigen. Therefore, the present invention has been completed based on these facts.

Therefore, the present invention is directed to providing a vaccine composition including gold nanoparticles; double-stranded DNA that is bound to the surfaces of the gold nanoparticles through a residue containing one or more functional groups and autonomously expresses an antigen within cells.

### [Technical Solution]

In order to solve the above problems, according to an aspect of the present invention, there is provided a vaccine composition including gold nanoparticles; double-stranded DNA that is bound to the surfaces of the gold nanoparticles through a residue containing one or more functional groups and autonomously expresses an antigen within cells.

Hereinafter, the present invention will be described in detail.

The present invention relates to a vaccine composition including gold nanoparticles, and double-stranded DNA that is bound to the surfaces of the gold nanoparticles via one or more thiolated residues and autonomously expresses an antigen within cells.

The double-stranded DNA of the present invention is intended to be introduced into cells and expressed. The type of double-stranded DNA is not limited, but may specifically include cDNA, gDNA, plasmid DNA, and PCR DNA.

The double-stranded DNA is bound to gold nanoparticles in a double-stranded form to deliver the double-stranded DNA into cells. This is different from the case in which the single-stranded DNA hybridizes with a sequence complementary to the single strand after it is bound to gold nanoparticles and introduced into cells. In particular, the double-stranded DNA may be derived from a gene capable of expressing an antigen. The antigen-expressing gene may be derived from a viral, bacterial, or cancer cell.

Also, since the double-stranded DNA includes one or more genes, the double-stranded DNA may be autonomously expressed after being introduced into cells. In the present invention, the term "autonomously expressed" means that the double-stranded DNA is autonomously transcribed and/or translated without being integrated into the genome of the injected cell. For autonomous expression, the double-stranded DNA may include one or more promoters, open reading frames, or terminators, but the present invention is not limited thereto. Also, the double-stranded DNA may be transcribed and/or translated to produce transcription and/or translation products, but the present invention is not limited thereto.

The vaccine composition of the present invention includes a gene delivery system in which the double-stranded DNA is bound to the surfaces of gold nanoparticles. The double-stranded DNA contains one or more functional groups for binding to the surfaces of the gold nanoparticles. The functional group may be a thiol group or an amine group, and may be included in one or more residues of the double-stranded DNA. In one embodiment of the present invention, the double-stranded DNA includes a thiolated residue, and then directly binds to the surfaces of the gold nanoparticles through the thiolated residue. The thiolated residue may be one or more bases at the 3' terminus, the 5' terminus, or within a base sequence of the double-stranded DNA.

Also, one or more double-stranded DNAs are bound to the surfaces of the gold nanoparticles. In this case, 1 to 20 identical or different double-stranded DNAs may be bound to the surfaces of the gold nanoparticles for expression, but the present invention is not limited thereto. Also, the size of the double-stranded DNA to be bound is not limited, but the double-stranded DNA may have a size of 10 bp or more, 100 bp or more, or 500 bp or more.

In one embodiment of the present invention, the double-stranded DNA is a SARS-CoV vaccine-derived RBD DNA sequence having a size of 720 bp, or a human papillomavirus (HPV)-derived DNA sequence having a size of 1,500 bp or more, but is not limited to the above types.

In the present invention, the term "vaccine" refers to a biological preparation containing an antigen that provides immunity to a subject, that is, an immunogenic or antigenic material that is administered (e.g., injected or orally administered) to a human or an animal to generate immunity in order to prevent a disease.

The vaccine of the present invention may be a DNA vaccine. The term "DNA vaccine" refers to a vaccine that induces an immune response by artificially replicating some of the genes of pathogens, viruses, and the like, and then administering the replicated genes. Such a DNA vaccine has various advantages over existing protein vaccines: i) since it may be synthetically produced using only the genetic information of a pure target pathogen antigen, there is no need to directly handle a dangerous pathogen, ii) since only some of the genes necessary for inducing toxicity are used, there is no concern about exhibiting any particular toxicity even when it is administered to a subject, and iii) because of its simplicity of being composed of only DNA, it is possible to develop a vaccine to respond quickly to various infectious diseases that occur suddenly.

The vaccine composition may develop immunity against various infectious diseases or cancers. The vaccine composition may be injected into a subject in various forms. The "injection" may be performed by any one method selected from the group consisting of subcutaneous injection, intramuscular injection, intradermal injection, intraperitoneal injection, nasal administration, oral administration, transdermal administration, and oral administration. More preferably, the vaccine composition may be administered by any route suitable for administration of a DNA vaccine, for example, subcutaneous, intramuscular, intraperitoneal, or intravenous injection.

Also, the vaccine composition may include one or more adjuvants and the like to improve or enhance the immune response. Suitable adjuvants may include a composition including a peptide, aluminum hydroxide, aluminum phosphate, aluminum oxide, a mineral or vegetable oil such as Marcol 52, and one or more emulsifiers, or a surface active substance such as lysolecithin, polyvalent cations, polyvalent anions, and the like.

In one embodiment of the present invention, the double-stranded DNA included in the vaccine composition may be used to encode an antigen including a protein, peptide, or fragment thereof of a coronavirus (SARS-CoV2). The coronavirus antigen may be a SARS-CoV2 spike protein, a SARS-CoV2 nucleocapsid protein, a SARS-CoV2 envelope protein, a SARS-CoV2 matrix protein, a fragment thereof, a variant thereof, or a combination thereof, but the present invention is not limited thereto. The antigen may be used to treat, prevent, and/or inhibit SARS-CoV-2-based pathology by preventing and treating any number of SARS-CoV2 strains.

The vaccine composition of the present invention significantly induces an immune response in a subject to which it is administered, and may induce both humoral and cellular immune responses. As a result, the vaccine composition of the present invention may have the effect of preventing and treating SARS-CoV-2 infection.

The gold nanoparticles included in the vaccine composition of the present invention are gold particles having a diameter in the nanometer range, preferably a diameter of 5 to 500 nm, more preferably 10 to 200 nm, and are easy to manufacture in the form of stable particles, easy to control the size thereof, and, unlike heavy metals such as manganese, aluminum, cadmium, lead, mercury, cobalt, nickel, beryllium, and the like, are harmless to the human body, and have high biocompatibility. When the diameter of the gold nanoparticles is larger than 500 nm, not only do their characteristics as nanoparticles disappear, but also the bond between the surface of gold, which has no nanomaterial characteristics, and a functional group such as a thiol group becomes weak, which makes it difficult to manufacture a carrier using the gold nanoparticles.

### [Advantageous Effects]

The present invention relates to a vaccine composition including double-stranded DNA delivered into cells via gold nanoparticles. More particularly, the double-stranded DNA expresses an antigen to induce an immune response, and thus can have preventive and therapeutic effects on various viral and bacterial infectious diseases and cancers.

### [Description of Drawings]

FIG. 1 shows a structure of a nucleic acid molecule including a gene of interest bound to gold nanoparticles according to one embodiment of the present invention.
FIG. 2 shows the results of confirming the binding efficiency of a nucleic acid molecule expressing SARS-CoV-2-RBD bound to the gold nanoparticles of the present invention through a thiolated residue.
FIG. 3 shows the results of delivering double-stranded DNA expressing SARS-CoV-2-RBD to cells to express the SARS-CoV-2-RBD.
FIG. 4 shows the results of confirming that SARS-CoV-2-RBD DNA bound to gold nanoparticles is *in vivo* injected and expresses an antigen.
FIG. 5 shows the results of confirming whether the SARS-CoV-2-RBD DNA bound to the gold nanoparticles is *in vivo* injected and produces a SARS-CoV-2 antibody.
FIG. 6 shows the results of confirming the binding efficiency of a nucleic acid molecule expressing HPV 16_L1 and HPV 18_L1 bound to the gold nanoparticles of the present invention through a thiolated residue.
FIG. 7 shows the results of confirming whether the double-stranded DNA of HPV 16_L1 and HPV 18_L1 is delivered into cells and expressed.
FIG. 8 shows the results of confirming whether the HPV 16_L1 and HPV 18_L1 DNA bound to the gold nanoparticles are *in vivo* injected and produce an antibody.

### [Best Mode]

Hereinafter, examples will be described in detail to specifically explain the present specification. However, it should be understood that the examples according to the present specification may be modified into various different forms, and the scope of the present specification is not intended to be limited to the examples described below. The examples of the present specification are provided to more completely explain the present specification to a person having ordinary skill in the art.

### Example 1: Manufacture of gold nanoparticles to which dsDNA (SARS-CoV2) is bound

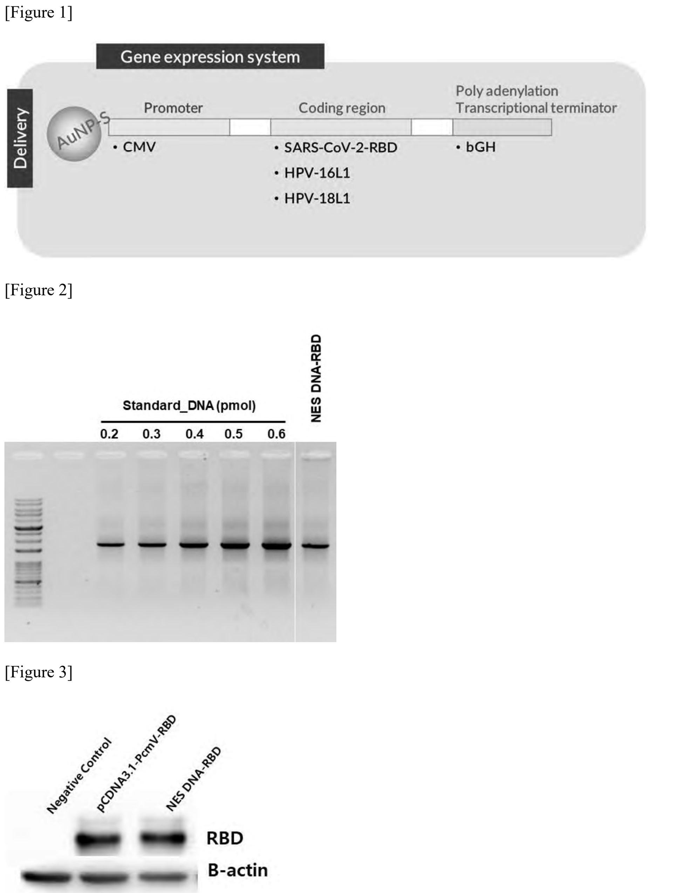

Double-stranded DNA derived from a coronavirus (SARS-CoV2) was bound to gold nanoparticles to produce NES DNA capable of intracellular delivery and expression. A schematic diagram thereof is shown in FIG. 1.

### 1-1. Manufacture of dsDNA capable of intracellular antigen expression

Using plasmid pcDNA3.1, a target gene for delivery was cloned between the CMV promoter and the bGH terminator of the plasmid. Each thiolated residue of the gene was subjected to PCR using a thiolated primer to synthesize double-stranded DNA in which the 3' terminus, the 5' terminus, or an internal residue was thiolated.

The double-stranded DNA used in the synthesis is a sequence (SEQ ID NO: 1) encoding an RBD domain of the spike protein of SARS-CoV2(δ). As the target gene for delivery, the SARS-CoV2(δ) RBD gene of SEQ ID NO: 1 was cloned through the above method, and the thiolated double-stranded DNA was synthesized by the PCR method using a thiolated primer.

### 1-2. Pretreatment of thiolated double-stranded DNA

After the synthesized thiolated double-stranded DNA was dissolved in water to a final concentration of 1 µM, 20 µL of 3 M sodium acetate (pH 5.2) and 30 µL of 1 N dithiothreitol (DTT) were added to 150 µL of the thiolated double-stranded DNA, and the mixture was allowed to react at room temperature for 60 minutes. To remove DTT containing unwanted thiol molecules, 200 µL of ethyl acetate was added and mixed, and the supernatant was removed by centrifugation. This process was repeated three times. Thereafter, the thiolated double-stranded DNA was precipitated using an ethanol precipitation method.

### 1-3. Manufacture of double-stranded dsDNA functionalized with gold nanoparticles (NES DNA)

The thiolated double-stranded DNA precipitated by pretreatment in Example 1-2 above was dissolved in water, added to gold nanoparticles, and then bound by a salt aging method. Specifically, the thiolated double-stranded DNA was added to 7 nM of gold nanoparticles (AuNP:thiolated double-stranded DNA = 1:40) and mixed thoroughly. Thereafter, NaCl was added to a concentration of 0.1 M, and the mixture was mixed for 4 hours. After 4 hours, NaCl was added to a concentration of 0.2 M, and the mixture was mixed for 4 hours. After 4 hours, NaCl was added to a concentration of 0.3 M, and the mixture was mixed for 12 hours.

After 12 hours, the mixture of thiolated double-stranded DNA and gold was collected by centrifugation at approximately 10,000 × g for 20 minutes, and the unreacted double-stranded DNA in the supernatant was then removed. This process was repeated three times.

The final AuNP-thiolated double-stranded DNA complex (AuNP-thiolated dsDNA) was dispersed in a 10 mM sodium phosphate buffer (pH 7.4) containing 0.1 M NaCl. The prepared AuNP-thiolated double-stranded DNA complex was analyzed by electrophoresis on a 1% agarose gel. As a result, it was confirmed that 10 to 15 thiolated double-stranded DNAs were bound per gold nanoparticle (FIG. 2).

### Example 2: Confirmation of in vitro protein expression level by RBD gene

This experiment was conducted to confirm the intracellular expression of the double-stranded DNA manufactured in Example 1 above. First, HeLa cells were transfected with plasmid DNA and the thiolated double-stranded DNA of Example 1 using Lipofectamine 3000 (Invitrogen). After 24 hours, the cells were washed with 1X PBS, collected using a scraper, and then lysed with an NP-40 lysis buffer (50 mM Tris-HCl (pH 8.0), 150 mM NaCl, 1% NP-40) containing a 1X protease inhibitor cocktail (GenDEPOT).

Next, after centrifugation, the concentration of total protein in the supernatant of the sample was measured using a BCA assay kit (Thermo Scientific), and 30 µg of total protein per sample was mixed with a protein dye (6X standard, 0.3 M Tris-HCl (pH 6.8), 12% SDS, 60% glycerol, 0.6 M β-mercaptoethanol, 0.0025% bromophenol blue), and electrophoresed on a 12% SDS-PAGE gel. Thereafter, protein expression was analyzed by Western blotting.

As a result, it was confirmed that the thiolated double-stranded DNA normally expressed the RBD protein within cells (FIG. 3).

### Example 3: Confirmation of gene delivery and expression by NES DNA (SARS-CoV2 RBD δ) in small animal model

To confirm an ability of the double-stranded DNA (SARS-CoV2 RBD δ: NES DNA), which was bound to gold nano carriers and delivered into cells, to express an antigen, the following experiment was conducted.

NES DNA (SARS-CoV2 RBD δ) was injected into the gastrocnemius muscle of 6-week-old Balb/c mice (Central Lab Animal Inc, Korea). After 36 hours, the gastrocnemius muscle was isolated, fixed with 4% PFA, and cryopreserved by being sequentially placed in 1X PBS containing 10%, 20%, and 30% sucrose, respectively. The cryopreserved tissue was placed in an FSC22 OCT compound (Leica Microsystems), rapidly frozen, and then cryosectioned at a thickness of 20 µm.

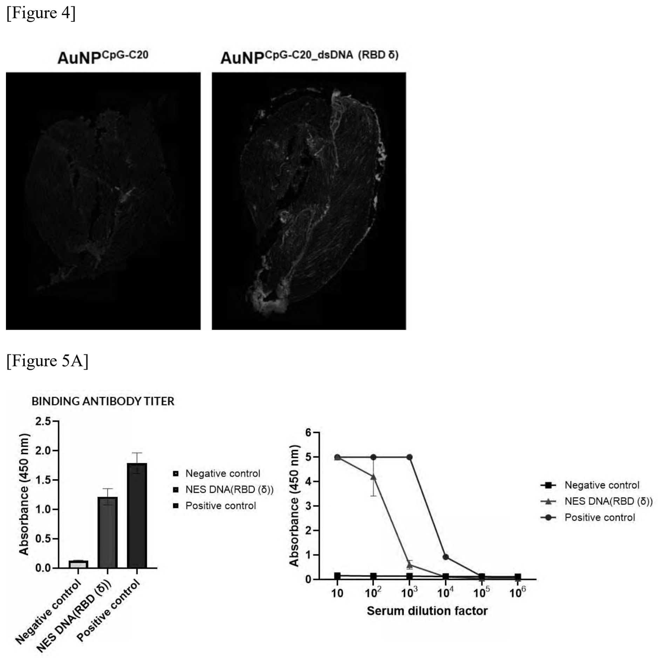

The protein expression in the sections was confirmed using a fluorescence microscope through immunofluorescence staining. As a result, as shown in FIG. 4, it was confirmed that the RBD δ protein was expressed in the gastrocnemius tissue injected with NES DNA (SARS-CoV2 RBD δ).

### Example 4: Confirmation of antibodies produced in vivo by NES DNA (SARS-CoV2 RBD δ)

An *in vivo* experiment was conducted to measure the concentration of a binding antibody titer and perform a competitive binding assay for detection of neutralizing antibody titer activity. First, Balb/c mice were vaccinated twice with an NES DNA (SARS-CoV2 RBD δ) vaccine at two-week intervals, and orbital blood was collected from the mice. Thereafter, the blood was allowed to coagulate at room temperature, and then centrifuged to separate the serum.

To measure binding antibodies to spike RBD δ produced in mouse blood, the separated serum was applied to a 96-well plate coated with the RBD protein and confirmed. The antibodies were detected by ELISA using a human ACE2 protein-coated 96-well plate and an HRP-bound RBD δ protein by performing competitive binding analysis of antibodies in the serum

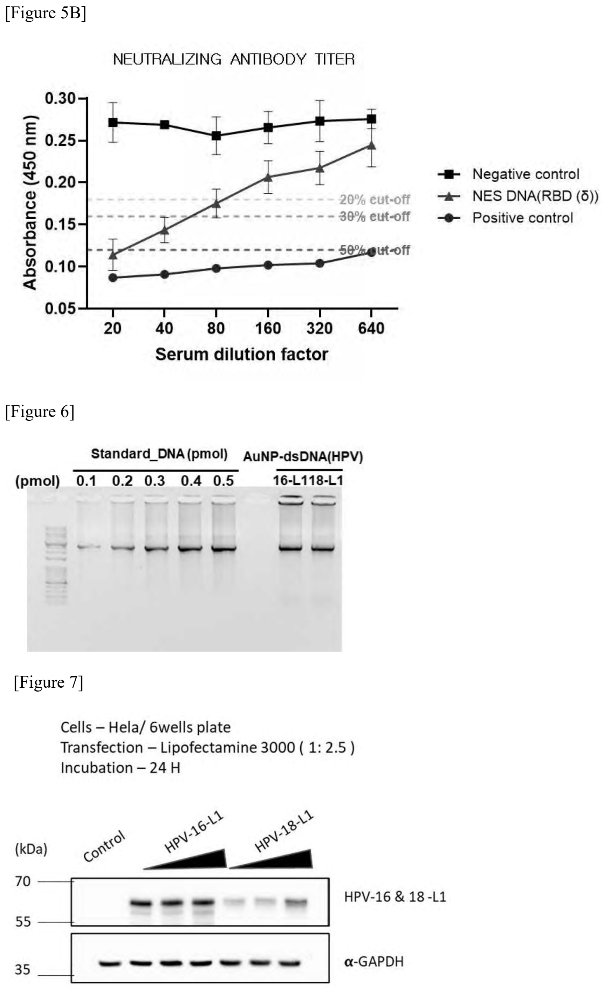

As a result, as shown in FIG. 5, it was confirmed that both binding antibodies and neutralizing antibodies to RBD were detected in the serum of mice inoculated with the NES DNA of the present invention.

### Example 5: Manufacture of gold nanoparticles to which double-stranded DNA (HPV) is bound

To determine whether intracellular delivery and expression are possible by binding human papillomavirus (HPV)-derived double-stranded DNA to gold nanoparticles, the following experiment was conducted.

First, HPV 16_L1 and HPV 18_L1, which are HPV virus-derived DNA sequences of SEQ ID NOS: 2 and 3, respectively, were synthesized (Table 2), and NES DNA-HPV 16_L1 and NES DNA-HPV 18_L1 were manufactured respectively in the same manner as in Example 1 by functionalizing the surfaces of gold nanoparticles. A schematic diagram thereof is as shown in FIG. 1.

As a result of confirming the binding efficiency of DNA bound to the surfaces of the gold nanoparticles, it was confirmed that10 to 15 thiolated double-stranded DNAs were bound per one gold nanoparticle in the case of the HPV-derived DNA (FIG. 6).

### Example 6: Confirmation of in vitro protein expression level by HPV16 L1 and HPV18 L1 genes

To confirm the intracellular expression of HPV 16_L1 and HPV 18_L1, which are double-stranded DNAs manufactured in Example 5 above, HeLa cells were transfected with a plasmid DNA and the thiolated double-stranded DNA using Lipofectamine 3000 (Invitrogen). The HPV16 L1 and HPV 18 L1 proteins expressed in the cells were analyzed by Western blotting using a 12% SDS PAGE gel.

As a result, as shown in FIG. 7, it was confirmed that the HPV16 L1 and HPV18 L1 proteins were expressed normally.

### Example 7: Confirmation of antibodies produced in vivo by NES DNAs (HPV16 L1, HPV18 L1)

To analyze the concentration of the binding antibody titer of the gold nanoparticle-bound DNA (NES DNA-HPV 16_L1, NES DNA-HPV 18_L1) synthesized in Example 5 above, the following experiment was conducted.

Balb/c mice were vaccinated twice with an NES DNA (HPV16 L1, HPV18 L1) vaccine at two-week intervals, and orbital blood was then collected from the mice. Thereafter, the blood was allowed to coagulate at room temperature, and then centrifuged to separate the serum.

The binding antibodies to HPV L1 produced in mouse blood were detected in the serum by ELISA using a 96-well plate coated with the HPV16 L1 VLP and HPV18 L1 VLP proteins.

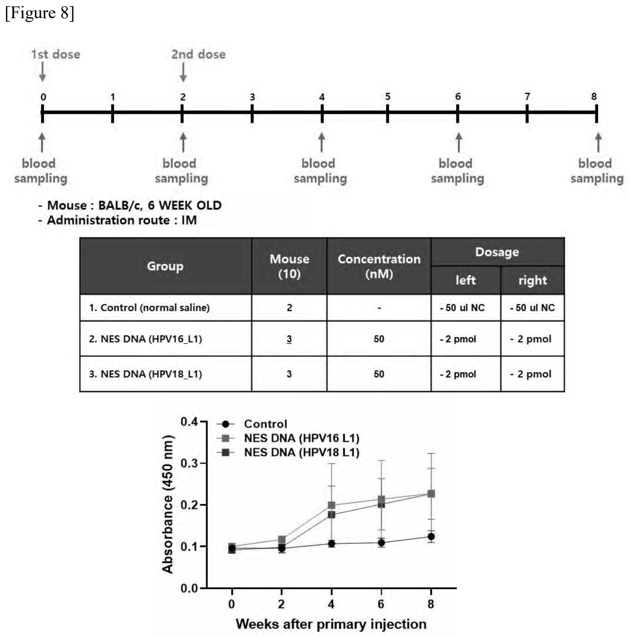

As a result, as shown in FIG. 8, it can be seen that the antibodies were formed in the mice administered the NES DNA (HPV16 L1, HPV18 L1) vaccine compared to the non-administered group (control).

### [Mode for Invention]

In one aspect, the present invention relates to a vaccine composition including gold nanoparticles; and double-stranded DNA that is bound to the surfaces of the gold nanoparticles via a residue containing one or more functional groups and autonomously expresses an antigen within cells.

As an example, the antigen includes a viral, bacterial or cancer antigen.

As an example, the double-stranded DNA autonomously expresses the antigen without being integrated into the genome of the injected cell.

As an example, the double-stranded DNA is derived from the SARS-CoV2 virus.

As an example, the double-stranded DNA is derived from human papillomavirus (HPV).

As an example, the residue containing the functional groups contains a thiol group or an amine group.

As an example, the residue containing the functional groups is one or more residues included at the 3' terminus, the 5' terminus, or within a base sequence of the double-stranded DNA.

As an example, the gold nanoparticles have a size of 5 to 500 nm.

In another aspect, the present invention relates to a method of expressing a viral, bacterial or cancer antigen by delivering double-stranded DNA, which is bound to the surfaces of gold nanoparticles through a residue containing one or more functional groups, into cells.

## Claims

1. A vaccine composition comprising:
gold nanoparticles; and
double-stranded DNA that is bound to the surfaces of the gold nanoparticles through a residue containing one or more functional groups and autonomously expresses an antigen within cells.

2. The vaccine composition of claim 1, wherein the antigen includes a viral, bacterial or cancer antigen.

3. The vaccine composition of claim 1, wherein the double-stranded DNA autonomously expresses the antigen without being integrated into the genome of the injected cell.

4. The vaccine composition of claim 1, wherein the double-stranded DNA is derived from the SARS-CoV2 virus.

5. The vaccine composition of claim 1, wherein the double-stranded DNA is derived from human papillomavirus (HPV).

6. The vaccine composition of claim 1, wherein the residue containing the functional groups contains a thiol group or an amine group.

7. The vaccine composition of claim 1, wherein the residue containing the functional groups is one or more residues included at the 3' terminus, the 5' terminus, or within a base sequence of the double-stranded DNA.

8. The vaccine composition of claim 1, wherein the gold nanoparticles have a size of 5 to 500 nm.

9. A method of expressing a viral, bacterial or cancer antigen by delivering double-stranded DNA, which is bound to the surfaces of gold nanoparticles through a residue containing one or more functional groups, into cells.
